(19) Europäisches Patentamt
European Patent Office
Office européen des brevets

(11) Veröffentlichungsnummer: **0 550 383 B1**

(12) **EUROPÄISCHE PATENTSCHRIFT**

(45) Veröffentlichungstag der Patentschrift: **28.06.95**

(51) Int. Cl.6: **C07D 471/10**, A61K 31/435, //(C07D471/10,221:00,221:00)

(21) Anmeldenummer: **92710032.1**

(22) Anmeldetag: **24.11.92**

Die Akte enthält technische Angaben, die nach dem Eingang der Anmeldung eingereicht wurden und die nicht in dieser Patentschrift enthalten sind.

# (54) Kristalline fumarsaure Salze von 9,9-Alkylen-3,7-diazabicyclononan-Verbindungen enthaltende Arzneimittel.

(30) Priorität: **03.12.91 DE 4139763**

(43) Veröffentlichungstag der Anmeldung: **07.07.93 Patentblatt 93/27**

(45) Bekanntmachung des Hinweises auf die Patenterteilung: **28.06.95 Patentblatt 95/26**

(84) Benannte Vertragsstaaten: **AT BE CH DE DK ES FR GB GR IE IT LI LU NL PT SE**

(56) Entgegenhaltungen:
**EP-A- 0 103 833**
**EP-A- 0 306 871**
**EP-A- 0 389 984**
**FR-A- 2 331 347**
**FR-A- 2 375 235**

**EUROPEAN JOURNAL OF MEDICINAL CHEMISTRY.CHIMICA THERAPEUTICA. Bd. 12, Nr. 4, 1977, CHATENAY-MALABRY FR Seiten 302 - 305 U. H RLEIN ']ber unsymmetrisch N-substituierte Bispidine (3,7-Diazabicyclo(3.3.1)nonane) I'**

**Houben-Weyl, Band I/1, Seiten 369-378**

(73) Patentinhaber: **Kali-Chemie Pharma GmbH**
**Hans-Böckler-Allee 20**
**D-30173 Hannover (DE)**

(72) Erfinder: **Schön, Uwe**
**Föhrenkamp 12**
**W-3167 Burgdorf (DE)**
Erfinder: **Heitmann, Walter**
**Sonnenweg 11**
**W-3006 Burgwedel 1 (DE)**
Erfinder: **Mätzel, Uwe**
**Habichtshorst 9**
**W-3167 Burgdorf (DE)**

(74) Vertreter: **Lauer, Dieter, Dr.**
**c/o Solvay Deutschland GmbH**
**Hans-Böckler-Allee 20**
**D-30173 Hannover (DE)**

EP 0 550 383 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue kristalline fumarsaure Salze von N,N'-disubstituierten 9,9-Alkylen-3,7-diazabicyclo[3,3,1]nonan-Verbindungen der allgemeinen Formel I

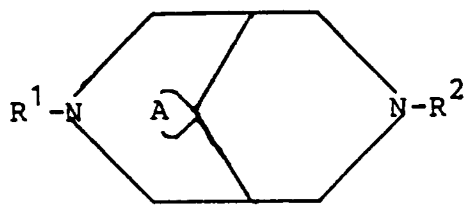

I

worin A eine Alkylenkette mit 4-5 Kohlenstoffatomen bedeutet und $R^1$ und $R^2$ unabhängig voneinander je eine geradkettige oder verzweigte Alkylgruppe mit 3-4 Kohlenstoffatomen oder die Cyclopropylmethylgruppe bedeuten.

Die 9,9-Alkylen-3,7-diazabicyclononan-Verbindungen der Formel I und ihre pharmakologischen Wirkungen sind aus dem europäischen Patent Nr. 10 38 33 und dem entsprechenden finnischen Patent Nr. 76 338 bekannt. Die Verbindungen der Formel I stellen eine Untergruppe der in den vorgenannten Patentschriften beschriebenen 9,9,N,N'-tetrasubstituierten 3,7-Diazabicyclo[3,3,1]nonan-Verbindungen dar und können nach den dort beschriebenen Methoden hergestellt werden. Aus den vorgenannten Patentschriften ist bekannt, daß die Verbindungen wertvolle herzwirksame Eigenschaften besitzen und insbesondere sauerstoffeinsparende, die Herzfrequenz beeinflussende und herzrhythmisierende Wirkungen zeigen und sich durch eine hohe physiologische Verträglichkeit auszeichnen. So zeigen die Verbindungen schon in geringen Dosen eine befriedigende antiarrhythmische Wirkung. Darüber hinaus ist eine unerwünschte negative Beeinflussung des Kontraktionsvermögens des Herzens äußerst gering. D.h. die Verbindungen weisen ein besonders günstiges Verhältnis von antiarrhythmischen, bzw. die Refraktärzeit des Herzens verlängernden Wirkungen zu negativ inotropen Nebenwirkungen auf. Wie in der am 15.06.1990 eingereichten deutschen Patentanmeldung Nr. P 40 19 080 beschrieben ist, besitzen die Verbindungen darüber hinaus auch eine ausgeprägte diuretische Wirkung mit einem günstigen Verhältnis zwischen Natrium- und Kaliumausscheidung.

Als Salze von Verbindungen der Formel I werden in dem finnischen Patent Nr. 76 388 das Dihydrogentartrat und in der nicht vorveröffentlichten deutschen Patentanmeldung Nr. P 40 19 080 das Dihydrogentartrat und das Dihydrochlorid des N,N'-Dicyclopropylmethyl-9,9-tetramethylen-3,7-diazabicyclo[3,3,1]nonan und das Dihydrogentartrat und das Dihydrogenfumarat des N-Isobutyl-N'-isopropyl-9,9-pentamethylen-3,7-diazabicyclo[3,3,1]nonan beschrieben.

Die bisher verwendeten Salze der Verbindungen der Formel I haben den Nachteil, daß sie nicht kristallin sondern wie z.B. die Hydrogentartrate amorph anfallen und/oder wie z.B. die Hydrochloride stark hygroskopisch sind. Bei derartigen Salzen sind wegen schwankender Lösungsmittelgehalte eine gleichbleibende stöchiometrische Zusammensetzung und eine gleichbleibende Bioverfügbarkeit nicht ohne besondere Vorsichtsmaßnahmen zu gewährleisten.

Aufgabe der Erfindung war es, kristalline und stöchiometrisch einheitliche, praktisch solvatfreie und nicht hygroskopische, jedoch in Wasser ausreichend lösliche Salze von 9,9,N,N'-tetrasubstituierten 3,7-Diazabicyclo[3,3,1]nonan-Verbindungen zu entwickeln.

Es wurde nun gefunden, daß solche 9,9,N,N'-tetrasubstituierten 3,7-Diazabicyclo[3,3,1]nonan-Verbindungen, welche in 9,9-Stellung durch eine Alkylenkette mit 4-5 Kohlenstoffatomen substituiert sind, also eine Spirostruktur besitzen, und an den beiden Stickstoffatomen jeweils durch eine Alkylgruppe mit 3-4 Kohlenstoffatomen oder durch Cyclopropylmethyl substituiert sind, mit Fumarsäure in einem molaren Verhältnis von 1:1,5 stabile kristalline Salze bilden können.

Gegenstand der Erfindung sind dementsprechend fumarsaure Salze von 9,9-Alkylen-3,7-diazabicyclo-[3,3,1]nonan-Verbindungen der allgemeinen Formel I

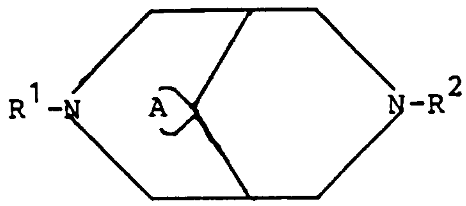

I

worin A eine Alkylenkette mit 4-5 Kohlenstoffatomen bedeutet und $R^1$ und $R^2$ unabhängig voneinander je eine geradkettige oder verzweigte Alkylgruppe mit 3-4 Kohlenstoffatomen oder die Cyclopropylmethylgruppe bedeuten, welche 1,5 Mol Fumarsäure pro Mol Base der Formel I enthalten.

Als Verbindungen der Formel I werden insbesondere N-Isobutyl-N'-isopropyl-9,9-pentamethylen-3,7-diazabicyclo[3,3,1]nonan (generic name = Bertosamil) und N,N'-Dicyclopropylmethyl-9,9-tetramethylen-3,7-diazabicyclo[3,3,1]nonan(generic name = Tedisamil) eingesetzt.

Die Herstellung der erfindungsgemäßen Sesquifumarate der Verbindungen der Formel I erfolgt, indem man eine Lösung der Base der Formel I und einer mindestens 1,5-fachen Molmenge an Fumarsäure in einem niederen Alkohol mit einem weniger polaren organischen Lösungmittel mischt, wobei das 1,5 Mol Fumarsäure pro Mol Base der Formel I enthaltende Salz kristallisiert, und das kristallisierte Salz abtrennt.

Als niedere Alkohole eignen sich z.B. Methanol, Ethanol oder Isopropanol, insbesondere Methanol oder Ethanol. Als im Vergleich mit dem niederen Alkohol weniger polares Lösungsmittel sind niedere aliphatische Ketone, insbesondere Aceton, oder niedere aliphatische offenkettige Ether, insbesondere Diethylether, geeignet.

Zweckmäßig wird eine im Verhältnis zur Base der Formel I 1,5-molare bis 2-molare Menge Fumarsäure eingesetzt. Falls ein Überschuß Fumarsäure eingesetzt wird, kann es, um Dihydrogenfumarat-Salz-Nebenprodukte zu entfernen, gegebenenfalls nötig sein, das gefällte Salz mehrmals umzukristallisieren, bis ein konstantbleibender Schmelzpunkt erreicht ist. Die Konzentration der Base der Formel I in der alkoholischen Lösung kann je nach Löslichkeit der Verbindung, der Lösetemperatur und der Ansatzmenge beliebig variiert werden und kann z.B. in Bereichen zwischen 3 und 50 Mol/l liegen.

Das Volumenverhältnis von alkoholischer Lösung und weniger polarem Lösungmittel kann je nach Konzentration der Verbindung der Formel I in der alkoholischen Lösung und der Löslichkeit des auszukristallisierenden Sesquifumarates der Verbindung der Formel I und der Art des weniger polaren Lösungsmittels variieren, wobei das Volumen an weniger polarem Lösungsmittel jedoch immer ein Mehrfaches des Volumen der alkoholischen Lösung beträgt. Als zweckmäßig erweist sich beispielsweise ein Volumenverhältnis von alkoholischer Lösung zu weniger polarem Lösungsmittel im Bereich von 1:15 bis 1:25, insbesondere ca. 1:20. Zur Vermischung der alkoholischen Lösung mit dem weniger polaren Lösungmittel kann entweder das weniger polare Lösungsmittel zu der alkoholischen Lösung zugesetzt werden, oder die alkoholische Lösung kann zu dem weniger polaren Lösungsmittel gegeben werden, z.B. allmählich zugetropft werden. Gewünschtenfalls kann das erhaltene Gemisch zur Bildung einer einheitlichen Lösung unter Rückfluß erhitzt werden, z.B. bis zum Siedepunkt des Reaktionsgemisches. Anschließend wird das Sesquifumarat der Verbindung der Formel I auskristallisieren gelassen, vorzugsweise unter Kühlung des Gemisches, z.B. bei Temperaturen zwischen Raumtemperatur und 0 °C.

Das auskristallisierte Salz kann auf an sich bekannte Weise von der Mutterlauge abgetrennt werden, z.B., gegebenenfalls unter vermindertem Druck, abfiltriert und anschließend bei leicht erhöhten Temperaturen, z.B. bei Temperaturen zwischen 40 und 65 °C, getrocknet werden, vorzugsweise in einem Vakuumtrockner.

Die nachfolgenden Beispiele sollen die Erfindung näher erläutern ohne jedoch ihren Umfang zu beschränken. Die Bestimmung der in den nachfolgenden Beispielen angegebenen Schmelzbereiche erfolgte mittels dynamischer Leistungsdifferenz-Kalorimetrie (= differential scanning calorimetry, Kurzbezeichnung "DSC") mit einem DSC 7-Gerät der Firma Perkin Elmer. Die den Schmelzbereich wiederspiegelnden Peaks der Energiekurve wurden bei einer Aufheizgeschwindigkeit von 10 °C/Minute bestimmt. Die Bestimmung des in den Beispielen angegebenen Gewichtsverlustes beim Trocknen erfolgte mittels Differenz-Temperatur-Gravimetrie (= thermogravimetric analysis, Kurzbezeichnung "TGA") mit einem TGA 7-Gerät der Firma Perkin Elmer mit einer Aufheizgeschwindigkeit von 20 °C/Minute.

Beispiel 1:

Herstellung von N-Isobutyl-N'-isopropyl-9,9-pentamethylen-3,7-diazahicyclo[3,3,1]nonan-sesquifumarat (= Bertosamil-sesquifumarat)

6,2 kg (= 11,8 Mol) Bertosamil-dihydrogenfumarat wurden in 2,6 l Methanol bei einer Temperatur von 50 °C gelöst. Anschließend wurden 52 l Aceton zu der Lösung gegeben und das Reaktionsgemisch wurde 7 Stunden am Rückfluß erhitzt. Dann wurde das Reaktionsgemisch langsam auf Raumtemperatur abgekühlt. Die Mutterlauge wurde von dem gebildeten Niederschlag über eine Nutsche abgesaugt, und dieser wurde mit 1 l Aceton gewaschen und im Trockenschrank bei 50 °C getrocknet. Es wurden 3,6 kg Rohkristallisat mit einem Schmelzpunkt von 90 bis 95 °C erhalten. Dieses Rohkristallisat wurde erneut in 2 l Methanol bei 50 °C gelöst, und zu der Lösung wurden 42 l Aceton gegeben. Das Reaktionsgemisch wurde langsam unter Rühren auf Raumtemperatur abgekühlt. Die Mutterlauge wurde von dem gebildeten Niederschlag über eine Nutsche abgesaugt, und dieser wurde mit 1 l Aceton gewaschen und im Trockenschrank bei 50 °C getrocknet. Es wurden 2,82 kg Bertosamil-sesquifumarat mit einem Schmelzbereich von 104,5 bis 107,4 °C erhalten. Dieser Schmelzbereich änderte sich bei weiterem Umkristallisieren aus Methanol/Aceton nicht. Das durch Titration bestimmte Verhältnis von Base:Säure betrug 1:1,5. TGA: es wurde kein Gewichtsverlust beim Trocknen festgestellt.

Beispiel 2:

Herstellung von N,N'-Dicyclopropylmethyl-9,9-tetramethylen-3,7-diazabicyclo[3,3,1]nonan-sesquifumarat (= Tedisamil-sesquifumarat)

3,7 g (= 0,0128 Mol) Tedisamil wurden in 25 ml Ethanol gelöst. Zu der Lösung wurde eine Lösung von 2,98 g (= 0,0256 Mol) Fumarsäure in 30 ml Ethanol gegeben. Die gebildete homogene Lösung wurde langsam in 500 ml Diethylether eingetropft und das Reaktionsgemisch wurde zur Vervollständigung der Salzbildung im Kühlschrank abgekühlt. Anschließend wurde die Mutterlauge von dem gebildeten Kristallisat über eine Nutsche abgesaugt, und dieses wurde mit 20 ml Diethylether gewaschen und im Vakuumtrockenschrank bei 60 °C getrocknet. Es wurden 4,9 g Tedisamilsesquifumarat mit einem Schmelzbereich von 135,8 bis 136,9 °C erhalten. Das durch Titration bestimmte Verhältnis Base:Säure betrug 1:1,5. TGA: es wurde kein Gewichtsverlust beim Trocknen festgestellt.
Die Eigenschaften der erfindungsgemäßen Sesquifumarate der Beispiele 1 und 2 wurden mit denen anderer Salze der gleichen Basen verglichen.

Vergleichsbeispiele A-G:

Herstellung von Vergleichssalzen von Bertosamil und Tedisamil mit anderen Säuren.

A) Bertosamil-dihydrogenfumarat
6 kg (= 20,5 Mol) Bertosamil wurden in 14,4 l Ethanol gelöst. Zu der Lösung wurden 4,76 kg (= 41 Mol) feste Fumarsäure gegeben. Das Reaktionsgemisch wurde auf 80 °C erwärmt und bei dieser Temperatur 30 Minuten lang gerührt. Anschließend wurde das Reaktionsgemisch langsam unter Rühren auf Raumtemperatur abkühlen gelassen und noch weitere 30 Minuten im Eisbad bei 0 °C gerührt. Die Mutterlauge wurde von dem gebildeten Niederschlag über eine Nutsche abgesaugt, und dieser wurde mit 10 l eiskaltem Ethanol gewaschen und im Trockenschrank bei 35 °C getrocknet. Es wurden 5,1 kg Bertosamil-dihydrogenfumarat erhalten. Die DSC ergab einen Schmelzbereich von 90,2 bis 96,2 °C. Ein weiterer Peak trat im Temperaturbereich von 68 - 81 °C auf und ist auf das Austreten von Ethanol zurückzuführen. TGA: der Gewichtsverlust des Salzes beim Trocknen betrug 7,0 %. Das durch Titration bestimmte Verhältnis Base;Säure betrug 1:2,1.
B) Bertosamil-dihydrochlorid
5,4 g (= 0,0154 Mol) Bertosamil wurden in 20 ml Isopropanol gelöst. Zu der Lösung wurden 8,2 ml einer 3,8-normalen Lösung von Chlorwasserstoffsäure in Isopropanol unter Eiskühlung gegeben. Anschließend wurde das Isopropanol abdestilliert. Der Rückstand wurde in wenig Aceton aufgenommen und bis zur einsetzenden Kristallisation mit Diethylether versetzt. Die Mutterlauge wurde von dem Niederschlag über eine Nutsche abgesaugt, und dieser bei 60 °C im Vakuumtrockenschrank getrocknet. Es wurden 5 g Bertosamil-dihydrochlorid mit einem Schmelzbereich von 180,6 bis 183,7 °C erhalten. TGA: der Gewichtsverlust beim Trocknen betrug 2,0 %. Das durch Titration bestimmte Verhältnis Base:Säure

betrug 1:2,0.

C) Bertosamil-dihydrogentartrat

5,4 g (= 0,018 Mol) Bertosamil wurden in 10 ml Essigsäureethylester gelöst. Zu der Lösung wurde eine Lösung von 5,5 g (= 0,037 Mol) L(+)-Weinsaure in 20 ml Aceton gegeben. Nach Abdestillieren des Lösungsmittels wurden 8,4 g Bertosamil-dihydrogentartrat als amorpher Schaum isoliert. Schmelzbereich 189,2 bis 201,2 °C unter Zersetzung. Das durch Titration bestimmte Verhältnis Base:Säure betrug 1:1,8. TGA: der Gewichtsverlust beim Trocknen betrug 0,1 %.

D) Bertosamil-salicylat

10,7 g (= 0,037 Mol) Bertosamil wurden in 40 ml Diethylether gelöst. Zu der Lösung wurde eine Lösung von 5,12 g (= 0,037 Mol) Salicylsäure in 30 ml Diethylether gegeben und das Reaktionsgemisch wurde 30 Minuten gerührt. Die Mutterlauge wurde von dem ausgefallenen Niederschlag über eine Nutsche abgesaugt, und dieser wurde bei 60 °C im Vakuumtrockenschrank getrocknet. Es wurden 14,5 g Bertosamil-salicylat mit einem Schmelzbereich von 118,4 bis 119,5 °C erhalten. Das durch Titration bestimmte Verhältnis Base:Säure betrug 1:1,0. TGA: es wurde kein Gewichtsverlust beim Trocknen festgestellt.

E) Tedisamil-dihydrochlorid

2 g (= 0,0069 Mol) Tedisamil wurden in 5 ml Isopropanol gelöst. Zu dieser Lösung wurde unter Rühren eine Lösung von 0,53 g Chlorwasserstoffsäure in 5 ml Isopropanol gegeben. Nach der Salzbildung wurde Isopropanol durch Destillation entfernt und das als Rückstand verbleibende Tedisamil-dihydrochlorid wurde aus Aceton umkristallisiert und bei 60 °C im Vakuumtrockenschrank getrocknet. Es wurden 2 g Tedisamil-dihyrochlorid erhalten. Die DSC ergab einen Schmelzbereich unter Zersetzung bei 225,7 bis 240,4 °C. Das durch Titration bestimmte Verhältnis Base:Säure betrug 1:2,0. TGA: der Gewichtsverlust beim Trocknen betrug 0,2 %.

F) Tedisamil-dihydrogentartrat

3,7 g (= 0,0128 Mol) Tedisamil wurden in 15 ml Essigsäureethylester gelöst. Zu der Lösung wurde eine Lösung von 3,85 g (= 0,0256 Mol) L(+)-Weinsäure in 50 ml Aceton gegeben. Nach Abdestillieren des Lösungsmittels wurden 6 g Tedisamil-dihydrogentartrat als amorpher Schaum isoliert. Die DSC ergab keinen definierten Schmelzbereich sondern Zersetzung ab 183 °C. TGA: der Gewichtsverlust beim Trocknen betrug 0,5 %. Das durch Titration bestimmte Verhältnis Base:Säure betrug 1:2,1.

G) Tedisamil-salicylat

10,7 g (= 0,037 Mol) Tedisamil wurden in 50 ml Diethylether gelöst. Zu der Lösung wurde eine Lösung von 5,12 g (= 0,037 Mol) Salicylsäure in 50 ml Diethylether gegeben. Das Reaktionsgemisch wurde 30 Minuten gerührt. Die Mutterlauge wurde von dem entstandenen Niederschlag über eine Nutsche abgesaugt, und dieser wurde im Vakuumtrockenschrank bei 60 °C getrocknet. Es wurden 14,5 g Tedisamil-salicylat erhalten. Die DSC ergab einen Schmelzbereich von 140,9 bis 142,2 °C. Das durch Titration bestimmte Verhältnis Base:Säure betrug 1:1,0. TGA: der Gewichtsverlust beim Trocknen betrug 0,1 %.

Die Eigenschaften der erfindungsgemäßen Salze gemäß den Beispielen 1 und 2 und der Vergleichssalze gemäß Vergleichsbeispielen A bis G wurden nach den folgenden Methoden bestimmt.

I. Bestimmung der Wasserlöslichkeit.

Die Wasserlöslichkeit wurde bei Raumtemperatur bestimmt.

II. Bestimmung des Lösungsmittelrestgehaltes.

Die Bestimmung von Lösungsmittelrestgehalten wurde mittels Kapillargaschromatographie durchgeführt unter Verwendung eines Sicromat $^{R}$-Gerätes der Firma Siemens mit Flammenionisationsdetektor.

III. Bestimmung der Hygroskopizität.

Zur Bestimmung der Hygroskopizität wurden Proben der Salze jeweils bei einer relativen Luftfeuchtigkeit von 55, 65, 76, 86, 92 und 100 % bei Raumtemperatur bis zur Gewichtskonstanz gelagert. Das Anfangsgewicht der Proben und das Gewicht nach Lagerung wurden gravimetrisch bestimmt und die Gewichtsdifferenz berechnet. Die Hygroskopizität der Substanzen wird als % Gewichtszunahme bezogen auf das Anfangsgewicht angegeben. Bei Substanzen mit Lösungsmittelgehalt wurde außerdem eine iodometrische Wassergehaltsbestimmung nach der Karl-Fischer-Methode durchgeführt.

Die nach den vorstehend beschriebenen Versuchsmethoden erhaltenen Ergebnisse sind in der nachfolgenden Tabelle wiedergegeben. Aus dieser Tabelle ist ersichtlich, daß die erfindungsgemäßen Sesquifumarate im Gegensatz zu den Vergleichssalzen praktisch nicht hygroskopisch sind und trotzdem eine für pharmazeutische Zwecke ausreichende Löslichkeit in Wasser besitzen.

Tabelle

| Salz Bsp. Nr. | $H_2O$-Löslichkeit in g/100 ml | Lösungsmittel-* restgehalt (GC) in % Gew. | | | | Hygroskopizität in % Gew. Zunahme bei einer relativen Luftfeuchtigkeit von | | | | | |
|---|---|---|---|---|---|---|---|---|---|---|---|
| | | Et | Ac | Di | Pr | 55 % | 65 % | 76 % | 86 % | 92 % | 100 % |
| 1 | 4,5 | - | - | - | - | 0 | 0 | 0,04 | 0,04 | - | 0,06 |
| A** | 3,6 | 7,8 | - | - | - | - 2,3 (+2,1 $H_2O$) | - 4,1 | - 4,2 | - 4,2 | - | - 4,0 (+ 3,5 $H_2O$) |
| B | > 51 | - | - | - | 0,03 | 7,7 | 9,0 | 13 | 51 | - | 171 |
| C | 50 | 0,7 | 1,8 | 0,02 | - | 4,3 | 7,0 | 9,6 | 15 | - | 102 |
| D | 1,6 | - | - | - | - | 0,1 | 3,1 | 14 | 17 | - | 35 |
| 2 | 4,4 | 0,12 | - | 0,05 | - | 0 | 0 | 0 | 0 | - | 1,1 |
| E | > 190 | - | - | - | 0,1 | 0,1 | 30 | 43 | 67 | 106 | - |
| F | 39 | 0,9 | 0,3 | - | - | 2,5 | 6,0 | 10 | 16 | - | 118 |
| G | 1,8 | - | - | 0,09 | - | 0 | 0,04 | 0,04 | 0,2 | - | 29 |

* Et=Ethanol, Ac=Aceton, Di=Diethylether, Pr=Isopropanol

** fällt als Solvat an, während der Lagerung findet eine Gewichtsabnahme durch Austausch von Solvens gegen Luftfeuchtigkeit statt. Dies wird durch die angegebenen - Werte wiedergespiegelt. In Klammern wird die nach der Karl-Fischer-Methode bestimmte Menge des von der Probe aufgenommenen Wassers angegeben.

## Patentansprüche

**1.** Fumarsaure Salze von 9,9-Alkylen-3,7-diazabicyclo [3,3,1]nonan-Verbindungen der allgemeinen Formel I

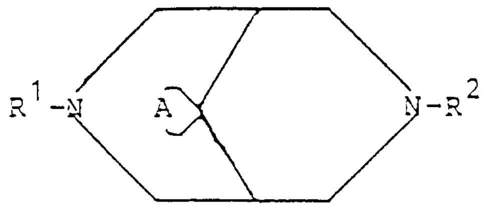

I

worin A eine Alkylenkette mit 4-5 Kohlenstoffatomen bedeutet und $R^1$ und $R^2$ unabhängig voneinander je eine geradkettige oder verzweigte Alkylgruppe mit 3-4 Kohlenstoffatomen oder die Cyclopropylmethylgruppe bedeuten, welche 1,5 Mol Fumarsäure pro Mol Base der Formel I enthalten.

**2.** Fumarsaure Salze gemäß Anspruch 1, worin die Base der Formel I N,N'-Dicyclopropylmethyl-9,9-tetramethylen-3,7-diaza-bicyclo[3.3.1]nonan oder N-Isobutyl-N'-isopropyl-9,9-pentamethylen-3,7-diazabicyclo[3,3,1]nonan darstellt.

**3.** N,N'-Dicyclopropylmethyl-9,9-tetramethylen-3,7-diazabicyclo[3.3.1]nonan-sesquifumarat gemäß Anspruch 2.

**4.** N-Isobutyl-N'-isopropyl-9,9-pentamethylen-3,7-diazabicyclo[3,3,1]nonan-sesquifumarat gemäß Anspruch 2.

**5.** Arzneimittel enthaltend eine pharmakologisch wirksame Menge eines fumarsauren Salzes gemäß Anspruch 1 und übliche pharmazeutische Hilfs- und/oder Trägerstoffe.

**6.** Verfahren zur Herstellung von fumarsauren Salzen von 9,9-Alkylen-3,7-diazabicyclo[3.3.1]nonan-Verbindungen der allgemeinen Formel I gemäß Anspruch 1

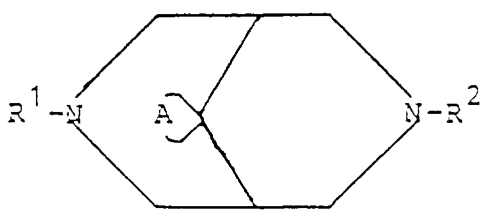

I

worin A, $R^1$ und $R^2$ die in Anspruch 1 angegebene Bedeutung besitzen, dadurch gekennzeichnet, daß eine Lösung der Base der Formel I und einer mindestens 1,5-fachen Molmenge an Fumarsäure in einem niederen Alkohol mit einem weniger polaren organischen Lösungsmittel gemischt wird, wobei das 1,5 Mol Fumarsäure pro Mol Base der Formel I enthaltende Salz kristallisiert, und das kristallisierte Salz abgetrennt wird.

**7.** Verfahren nach Anspruch 6, dadurch gekennzeichnet, daß als weniger polares Lösungsmittel Aceton oder Diethylether einsetzt werden.

## Claims

1. Fumaric acid salts of 9,9-alkylene-3,7-diazabicyclo[3.3.1]nonane compounds of the general formula I

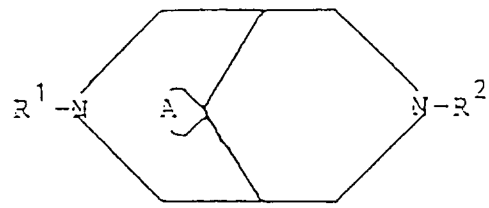

I

in which A denotes an alkylene chain of 4-5 carbon atoms and $R^1$ and $R^2$ independently of one another each denote a straight-chain or branched alkyl group of 3-4 carbon atoms or the cyclopropylmethyl group, which contain 1.5 mol of fumaric acid per mole of base of the formula I.

2. Fumaric acid salts according to Claim 1, in which the base of the formula I is N,N'-dicyclopropylmethyl-9,9-tetramethylene-3,7-diazabicyclo[3.3.1]nonane or N-isobutyl-N'-isopropyl-9,9-pentamethylene-3,7-diazabicyclo[3.3.1]nonane.

3. N,N'-Dicyclopropylmethyl-9,9-tetramethylene-3,7-diazabicyclo[3.3.1]nonane-sesquifumarate according to Claim 2.

4. N-Isobutyl-N'-isopropyl-9,9-pentamethylene-3,7-diazabicyclo[3.3.1]nonane-sesquifumarate according to Claim 2.

5. Medicament containing a pharmacologically active amount of a fumaric acid salt according to Claim 1 and customary pharmaceutical auxiliaries and/or excipients.

6. Process for the preparation of fumaric acid salts of 9,9-alkylene-3,7-diasabicyclo[3.3.1]nonane compounds of the general formula I according to Claim 1

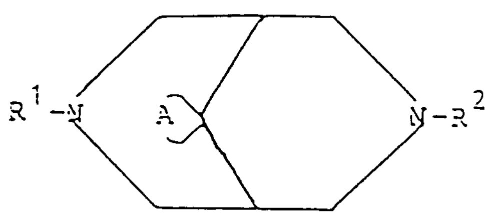

I

in which A, $R^1$ and $R^2$ have the meanings given in Claim 1, characterised in that a solution of the base of the formula I and of an at least 1.5-fold molar amount of fumaric acid in a lower alcohol is mixed with a less polar organic solvent, whereby the salt containing 1.5 mol of fumaric acid per mole of base of the formula I crystallises and the crystallised salt is separated off.

7. Process according to Claim 6, characterised in that the less polar solvent employed is acetone or diethyl ether.

## Revendications

1. Sels de l'acide fumarique de 9,9-alkylène-3,7-diazabicyclo [3,3,1] nonanes, composés de formule générale I:

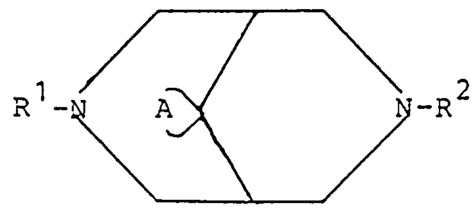

I

(dans laquelle A représente une chaîne alkylène ayant 4 ou 5 atomes de carbone; et $R^1$ et $R^2$ représentent chacun, indépendamment l'un de l'autre, un groupe alkyle linéaire ou ramifié ayant 3 ou 4 atomes de carbone ou le groupe cyclopropylméthyle), ces sels contenant 1,5 mole d'acide fumarique par mole de la base de formule I.

**2.** Sels d'acide fumarique selon la revendication 1, dans lesquels la base de formule I est le N,N'-dicyclopropylméthyl-9,9-tétraméthylène-3,7-diazabicyclo [3,3,1] nonane ou le N-isobutyl-N'-isopropyl-9,9-pentaméthylène-3,7-diazabicyclo [3,3,1] nonane.

**3.** Le sesquifumarate de N,N'-dicyclopropylméthyl-9,9-tétraméthylène-3,7-diazabicyclo [3,3,1] nonane selon la revendication 2.

**4.** Le sesquifumarate de N-isobutyl-N'-isopropyl-9,9-pentaméthylène-3,7-diazabicyclo [3,3,1] nonane selon la revendication 2.

**5.** Médicament contenant une quantité pharmacologiquement active d'un sel de l'acide fumarique selon la revendication 1 et des adjuvants et/ou excipients ou supports pharmaceutiques habituels.

**6.** Procédé pour préparer des sels de l'acide fumarique de 9,9-alkylène-3,7-diazabicyclo [3,3,1] nonanes, composés répondant à la formule générale I selon la revendication 1,

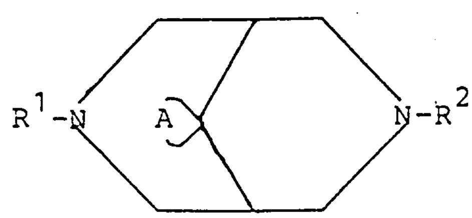

I

(dans laquelle A, $R^1$ et $R^2$ ont le sens indiqué à la revendication 1), procédé caractérisé en ce qu'on mélange une solution de la base de formule I et une quantité au moins 1,5 fois molaire d'acide fumarique dans un alcool inférieur avec un solvant organique moins polaire, par quoi le sel contenant 1,5 mole d'acide fumarique par mole de la base de formule I cristallise, et l'on sépare le sel cristallise.

**7.** Procédé selon la revendication 6, caractérisé en ce qu'on utilise, comme solvant moins polaire, l'acétone ou l'éther diéthylique.